# EUROPEAN PATENT SPECIFICATION

(11) **EP 4 620 381 B1**
(45) Date of publication and mention of the grant of the patent: **02.12.2026**
(21) Application number: 24164956.5
(22) Date of filing: 20.03.2024
(51) Int. Cl.: A61B 5/00, A61B 8/00

(54) **SYSTEM FOR LOCALISING AND MEASURING LIGHT IN LIGHT-SCATTERING MEDIA**
SYSTEM ZUR LOKALISIERUNG UND MESSUNG VON LICHT IN LICHTSTREUENDEN MEDIEN
SYSTÈME DE LOCALISATION ET DE MESURE DE LUMIÈRE DANS UN MILIEU DIFFUSANT LA LUMIÈRE

(43) Date of publication of application: 24.09.2025
(73) Proprietor: Deep Light Vision AB, 222 29 Lund (SE)
(72) Inventor: KINOS, Adam, 245 63 Hjärup (SE); RIPPE, Lars, 226 48 Lund (SE); KRÖLL, Stefan, 247 94 Dalby (SE)
(74) Representative: AWA Sweden AB - Alternative

(56) References cited:
- WO-A1-2023/067198
- WO-A1-2023/099705
- US-A1- 2009 066 949

## Description

### BACKGROUND OF THE INVENTION

### Field of the Invention

This disclosure pertains in general to a device and method to selectively attain light deep inside a light scattering medium, such as biological tissue. More particularly, the disclosure relates to a device and method to achieve more localized measurements using light deep inside a scattering medium than can be achieved by conventional methods. Especially, the disclosure relates to the use of acoustic-frequency-shifted laser light and optical components to achieve localized light measurements deep inside a scattering medium.

### Background of the Disclosure

The ability to shine light into turbid, i.e., light scattering, media is important in many settings, including but not limited to biomedical applications. Optical imaging provides highly sensitive molecular contrast in a simple and non-invasive manner. Traditional light microscopy relies on penetration of light through shallow layers, in the order of tens of micrometres. Confocal microscopy techniques extend the penetration depths up to in the order of 0.1 mm, while optical coherence tomography can reach to a depth in the order of 0.5 mm. This is approximately the limit of high-resolution optical imaging modalities, which rely on the suppression of diffusely scattered light. At deeper depths, diffuse light scattering dominates the interaction of the light field and the medium. The attenuation of an impending light beam at a certain depth is largely determined by the scattering of the medium. This fundamental aspect means that even at the most favourable wavelengths in the near-infrared wavelength region, the maximum penetration depth where light is detectable is limited to in the order of 10 cm in typical biological tissue.

In the diffuse scattering regime, from about a millimetre up to several centimetres, other optical analysis and optical imaging modalities have been developed. One example is pulse oximetry for determination of blood oxygen saturation. One example of optical imaging is diffuse optical tomography, which can be performed, for example, with endogenous tissue contrast, with added contrast agents, in fluorescence, or with added fluorescing agents. Generally, because of the diffuse scattering, spatial localization is limited with these methods to in the order of several millimetres up to a centimetre or more. In addition, because of the very low light intensities in the detected light, the detection schemes are often cumbersome, slow, and require advanced and expensive equipment.

To improve the spatial localization when probing turbid media with light, photoacoustic schemes have been devised. In photoacoustic tomography, acoustic waves that emanate locally, due to slight heating in the tissue due to absorption of laser pulses, are detected by ultrasound transducers. Photoacoustic tomography takes advantage of the fact that ultrasound waves are scattered several orders of magnitude less than light waves in tissue. Thus, the spatial localisation of the origin of the acoustic waves can be in the order of a millimetre or less. The depth achievable in photoacoustic tomography is fundamentally limited by the acoustic background noise of the medium. This means that photoacoustic imaging in live human tissue is limited to a maximum of about 2 cm.

Another example of the use of acoustics to improve localisation of light interaction in tissue is the use of acoustic photon tagging, as described for example in J. Gunther & S. Anderson-Engels (2017, October), Review of current methods of acousto-optical tomography for biomedical applications, Frontiers of Optoelectronics, 10(3), 211-238. By insonifying tissue, and directing laser light into the tissue, only the laser light that passes through the region occupied by an acoustic field will be frequency-shifted by the acoustic frequency. By optical detection of the frequency-shifted light only, it can be known that that light has interacted with the acoustic field, which compared to the light can be made to occupy small and localized volume. This scheme is called ultrasound optical tomography (UOT). Unlike photoacoustic tomography, this scheme is not limited by the acoustic background noise, but instead is limited only by the ability to measure the optical signal. This allows imaging considerably deeper into tissue (>5 cm).

In UOT, one tries to detect small changes in the signal due to different optical properties of the tissue. However, the photon flux inside the tissue varies, thus giving a larger background variation. This difference in photon flux is normally compensated for so that it is easier to extract the small signal variations due to the optical properties of the tissue. In Kim et al, Sentinel lymph node detection ex vivo using ultrasound-modulated optical tomography, J Biomed Opt. 2008, 13(2) 020507, and in Kim et al, Ultrasound-modulated optical tomography in reflection-mode with ring-shaped light illumination, J Biomed Opt. 2009, 14(2) 024015, they use a ring-shaped light illumination scheme to provide a more even light illumination inside the tissue compared to previous light illumination techniques. A further system is known from WO 2023/067198 A1.

Although photoacoustic methods can provide better spatial resolution than pure optical methods, these methods still suffer from the limitation that the light does not easily penetrate deep into light-scattering media, due to the strong attenuation provided by the scattering.

As described above, previously known methods to perform light measurements inside a light-scattering medium have a limited penetration depth and/or poor spatial resolution. Hence, new improved apparatus and methods for deep light penetration in light scattering media would be advantageous.

### SUMMARY OF THE INVENTION

The invention is defined by the appended claims. Accordingly, examples of the present disclosure preferably seek to mitigate, alleviate or eliminate one or more deficiencies, disadvantages or issues in the art, such as the above-identified, singly or in any combination by providing a device, system or method according to the appended patent claims for selectively attaining high light intensities deep inside scattering media for the purpose of localised light measurements.

In a first aspect, the disclosure relates to a system for light measurements in a human or animal. The system comprising at least one ultrasound transducer and at least one light emitter for emitting light over an extended area within a wavelength-range of infrared (IR), such as Near infrared (NIR), visible or ultraviolet light. The system may further comprise at least one optical filter, at least one light detector and a processing unit.

The ultrasound transducer may be configured for directing at least one ultrasound field with at least one ultrasound frequency to at least one location inside a tissue site of said human or animal. The at least one light emitter may be configured for directing light from at least two positions, or by covering an extended area, with at least one light frequency into the tissue site of the human or animal.

The at least one optical filter may be arranged before the at least one light detector for suppressing the light frequency but transmitting light with at least one light frequency that is frequency-shifted by the ultrasound frequency.

The at least one light detector detecting the light that is frequency-shifted by the ultrasound frequency and the processing unit may process the detected frequency-shifted light from the at least two positions, or the extended area, to provide information of the at least one location inside the tissue site.

In one example, the at least one light emitter may be at least two light sources, such as lasers, arranged to direct light into the tissue site from different discrete positions.

In one example, the at least one light emitter may be a single light source extending over the extended area, wherein the single light source is arranged to direct light into the tissue site from the extended area.

In one example, the at least one light detector may be at least two light detector or a spatially resolved detector.

In one example, the at least one optical filter may be at least one crystal, and a separate crystal is arranged before each of the at least two light detectors, or wherein separate regions of a single crystal is associated with each of the at least two detectors or sections of the spatially resolve detector.

In one example, reflective tubes may be arranged to guide light to separate parts of the at least one crystal and from each of the separate parts to separate detectors of the at least two detectors or separate sections of the spatially resolved detector.

In one example, the processing unit may utilize information related to a position of the emitted light and a position of the detected frequency-shifted light to provide information of the at least one location inside the tissue site.

In one example, the processing unit may provide a reconstruction, such as an image, of the at least one location inside the tissue site.

In one example, the at least one ultrasound transducer may be configured to scan a 3D volume or be translated.

In one example, the at least one light emitter may be configured to emit light corresponding to at least two different wavelengths.

In one example, the system may further comprise at least one of time-of-flight, frequency-domain photon-migration spectroscopy, or diffuse optical tomography to provide optical properties of the tissue.

In one example, the optical properties are used to improve resolution and/or contrast.

In one example, the at least one light emitter may be stabilized to a certain wavelength or frequency by means of an external optical cavity, by locking the wavelength or frequency to a spectral hole in a crystal, or by locking the wavelength or frequency by using a fiber interferometer or fiber cavity.

In one example, a reference may be obtained by measuring on at least one separate location, or wherein a reference is obtained through simulations using diffusion equation or Monte Carlo methods, and wherein the reference is removed from the information of the at least one location inside the tissue site.

In one example, the information of the at least one location inside the tissue site may be obtained as a function of time and combined with bodily cycles to extract additional information.

It should be emphasized that the term "light" when used in this specification is not limited to light visible to the human eye, but also comprises wavelengths in the ultraviolet and infrared regions, such as Near infrared.

It should also be emphasized that the term "comprises/comprising" when used in this specification is taken to specify the presence of stated features, integers, steps or components but does not preclude the presence or addition of one or more other features, integers, steps, components or groups thereof.

### BRIEF DESCRIPTION OF THE DRAWINGS

These and other aspects, features and advantages of which examples of the disclosure are capable of will be apparent and elucidated from the following description of examples of the present disclosure, reference being made to the accompanying drawings, in which
Fig. 1 is illustrating example of schematic setup for imaging using acoustic photon tagging.
Fig. 2 is three experimentally obtained images of an ultrasound optical tomography (UOT) signal where the tissue-mimicking phantoms have similar optical properties as tissue and each image contain an inclusion with varying relative absorption coefficient relative the background tissue. The figure also shows ultrasound images for each experimental case. The UOT signal is measured using an example of a system of Fig. 1.
Fig. 3 is illustrating a schematic crystal with 3 different classes of doped ions. Such a crystal may be used to create a slow light filter.
Fig. 4 is illustrating a schematic slow-light filter. The absorption of the ion classes in the crystal have been manipulated to allow for passage of the frequency-shifted light (full) while the original laser frequency (dashed) is absorbed.
Fig. 5 is schematically illustrating the most likely paths photons will travel from the source (19) via the ultrasound focus (15) before leaving the tissue (20) and is detected.
Fig. 6 is schematically illustrating the case when using three sources and three detectors, indicating that all photon paths travel through the ultrasound focus (15), but otherwise can take widely different paths.
Fig. 7 is schematically illustrating the case when using an extended source and detector, again indicating that all photon paths travel through the ultrasound focus (15) but otherwise can take widely different paths (show through the colour gradient)
Fig. 8 is showing Monte Carlo simulation results of UOT signals (left graphs) using one source and detector (Fig. 8a) or multiple sources and detectors (Fig. 8b), as well as an illustration of the Jacobian (right graphs) for how the UOT signal at the black circle in the left graph is affected by the optical properties of the tissue.
Fig. 9 shows examples of how the absorption coefficient might vary as a function of wavelength for different tissue types (skin, adipose, glandular, and tumour) assuming specific concentrations of a few tissue chromophores.
Fig. 10 shows a compact design of how a locking crystal can be mounted inside a cryostat with ferrule sleeves and gradient-index lenses (GRIN).
Fig. 11 shows a flow-chart of an exemplary method for imaging using acoustic photon tagging.

### DESCRIPTION OF EXAMPLES

The following disclosure focuses on examples of the present disclosure applicable to improve localisation of light interaction in light-scattering media for the purpose of light measurements. The disclosure may be applicable for measurements of oxygen saturation deep inside living tissue, such as the brain, heart, female breast, or muscle tissue. However, it will be appreciated that the description is not limited to this application but may be applied to many other systems where localisation of light interaction in light-scattering media is useful.

In general terms, the disclosure concerns an optical part and an ultrasound part that are combined inside tissue and the optical part exiting the tissue is filtered through an optical filter before being detected and analysed by a processing unit. The application relates to the use of multiple or extended light sources, multiple or extended detectors, and using multiple or separate regions of a crystal as the optical filter. The optical light may be transferred via reflective tubes between the tissue and the crystal and from the crystal to the detector. Furthermore, the optical light may be frequency stabilised using either an external optical cavity or by locking the frequency to a spectral hole in a crystal. The disclosure also relates to how the processor unit may reconstructing tissue properties or extracting information from measurements by, e.g., using prior knowledge in the form of information from conventional medical techniques or from additional measurements performed using other ultrasound or optical methods. Furthermore, the disclosure relates to the use of multiple wavelengths of the optical light to better extract useful information regarding tissue constituents. The ultrasound part may also cover a 3D volume either through scanning or by translation, which is important when the measured information is used to reconstruct the tissue properties. The disclosure may also relate to how the reconstruction may be improved by taking reference measurements at different spatial locations or through the use of diffusion equation or Monte Carlo simulations. Further, the disclosure may relate to how the reconstruction or measurements might be improved by performing measurements over time and synchronizing the measurements with bodily cycles.

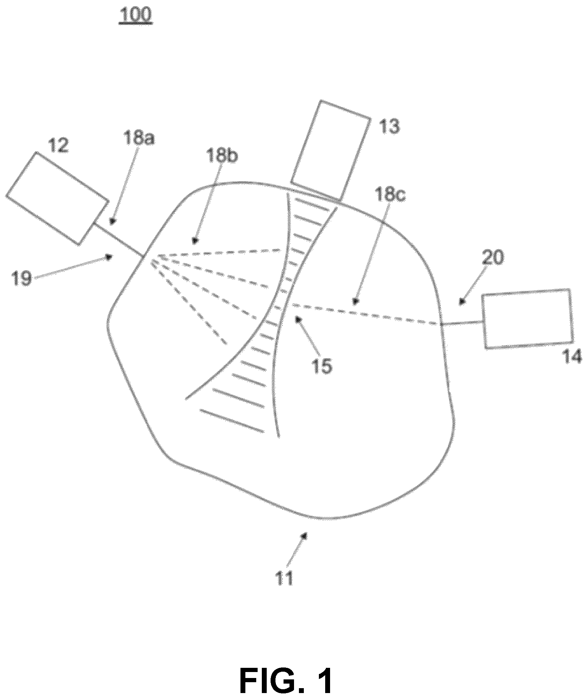

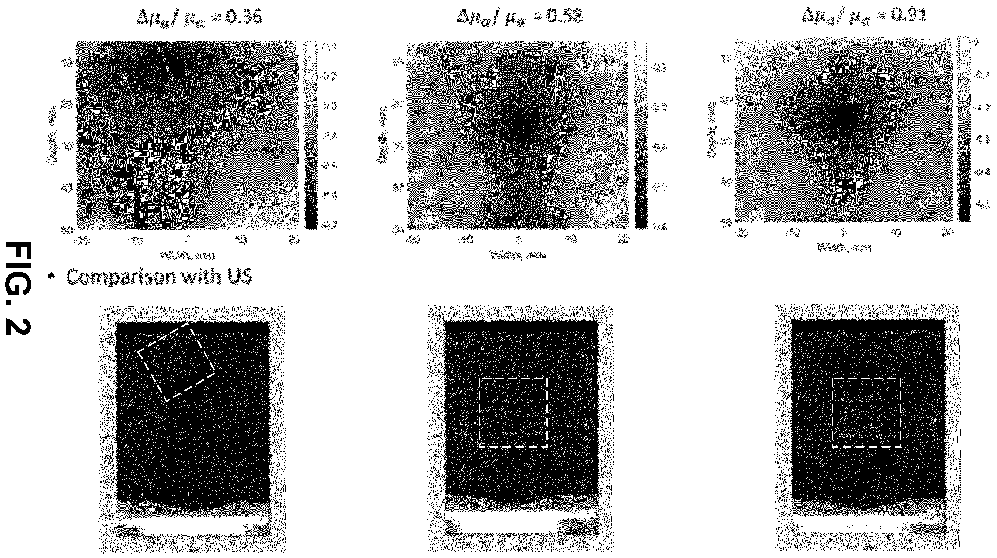

In an example, in the schematic illustration in Fig 1, the system 100 comprises an optical part of the system which includes at least one light source 12, such as a laser, and an optical filter 14. The at least one light source may be configured for emitting light directed from at least two positions or covering an extended area. An acoustic part of the system includes an ultrasonic device 13. The ultrasonic device 13 emits an acoustic field into the light-scattering medium 11 at in order to occupy the point 15. A light beam 18a emitted from the laser 12 is directed into the medium 11, and the light beam 18a is diffusely scattered 18b in the medium. Part of the diffused light 18b may pass through the position occupied by the ultrasound field 15, and may be frequency-shifted through interaction with the ultrasound field. The ultrasound frequency may be in the order of 10⁶ Hz, while the optical frequency may be in the order of 10¹⁴ Hz. Since the optical frequency of the laser light is many orders of magnitude higher than the frequency of the ultrasound, the frequency shift corresponds to a small wavelength difference in the laser light, which may be in the order of a few femtometers. The frequency-shifted light may propagate from the position occupied by the ultrasound field 15 diffusely through the medium, and part of the frequency-shifted light 18c may leave the medium and hit the optical filter 14. The optical filter 14 largely removes the original laser frequency, leaving substantially the frequency-shifted light, which can be detected. The optical filter 14 needs to have special properties to suppress the carrier frequency at around 10¹⁴ Hz, when the frequency-shifted light is only separated by in the order of 10⁶ Hz. It also needs to be largely independent of the angle of the incoming light, which is diffuse. This performance can be realised as a so-called slow-light filter, as described in H. Zang et al (2012, March), Slow light for deep tissue imaging with ultrasound modulation, Appl. Phys. Lett. 100(13), 131102. The frequency-shifted light carries information predominantly from the position 15, and thus this setup provides spatial information inside the medium 11. By scanning the position 15, representing the volume occupied by the ultrasound, it is possible to attain a map or image of the optical contrast inside the medium 11, such as information about the oxygen saturation in tissue. The lateral scanning may be performed either by mechanical movement of the ultrasound source or by controlling the individual elements of the ultrasound source to move the ultrasound field electronically. The longitudinal scanning is performed by timing an optical pulse to different depth positions of the ultrasonic pulse, or, according to the invention, by timing the light measurement to different depth positions of the ultrasonic pulse while illuminating the tissue with continuous wave (cw) light or quasi-cw light. An experimentally obtained image generated in this manner is seen in Fig. 2, where three experimentally obtained images of an ultrasound optical tomography (UOT) signal where the tissue-mimicking phantoms have similar optical properties as tissue and each image contain an inclusion with varying relative absorption coefficient relative the background tissue. The figure also shows ultrasound images for each experimental case. In the ultrasound images, a square with broken lines has been added the images to highlight the area of the inclusion.

In Fig. 2, the position 15 has been scanned over an area of high absorption, i.e. low oxygen saturation for wavelengths shorter than 800 nm. In order to extract information about the oxygen saturation, it is preferable to perform the measurement using at least two different wavelengths. For example, it is preferable to have one wavelength around 800 nm, and one wavelength in the interval 600-770 nm. The spectral absorption profiles of oxygen-saturated haemoglobin and non-oxygen-saturated haemoglobin are such that the light absorption is similar at around 800 nm, while different at wavelengths below or above 800 nm. Alternatively, one can choose one wavelength at around 800 nm, and another wavelength in the interval 820 - 1200 nm. By comparing the signal at the two wavelengths, it is possible to deduce the ratio of oxygen-saturated to non-oxygen-saturated haemoglobin. Note that the method is not limited to two wavelengths. In some situations, it may be preferable to use more than two wavelengths, e.g., to increase the accuracy when determining the oxygen saturation.

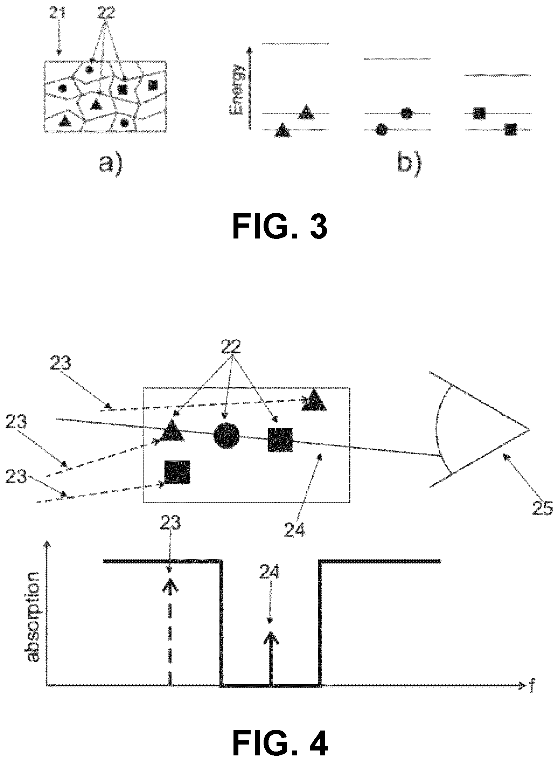

The optical filter 14 may be comprised of a slow-light filter, example given in Fig. 3 and 4. The slow-light effect is obtained by creating a narrowband spectral transmission window in an otherwise strongly absorbing background, which through the Kramers-Kronig relation leads to a strong dispersion and hence a slow group velocity, i.e., a slow light effect. This filter may be comprised of a host crystal 21 doped with ions 22 with strong optical absorption at the original frequency of the laser 23. As the ions replace other atoms in the host crystal, they may slightly distort its crystal lattice 21. While the individual absorption of these ions is strong and narrow in frequency, the entire ensemble of doped ions may see different crystal fields and thus slightly change at what frequency they absorb in relation to each other. In Fig. 3, three different ion classes 22 are depicted which have different energy separations between their ground and excited states, as seen in Fig. 3b). The multitude of different ion classes yield a total absorption profile of the ensemble of doped ions in the host crystal that may be two to three orders of magnitude wider in frequency than any frequency shift induced by the acoustic field.

In this absorption profile it may be possible to construct different enduring spectral structures by optical pumping techniques. This optical pumping may be performed with or without the application of electric and/or magnetic fields to split energy levels of the ions. An example of a resulting filter and the crystal absorption profile is seen in Fig. 4, where the absorption may be unaffected or higher for the original laser frequency light 23 while any absorption may be removed for the frequency-shifted light 24 which can proceed to a detector 25. A side effect of this modified absorption profile is that the frequency shifted light may experience a reduction in group velocity by several orders of magnitude. As such, the signal 24 may be additionally differentiated from the original laser light 23 via time gating.

The spatial information carried by the frequency-shifted light is predominantly from the location of the ultrasound field at 15, but since the light propagates from the light input location 19 via the ultrasound focus 15 to the output aperture 20, the frequency-shifted light will be affected by any absorption present along the propagation path from 19 via 15 to 20.

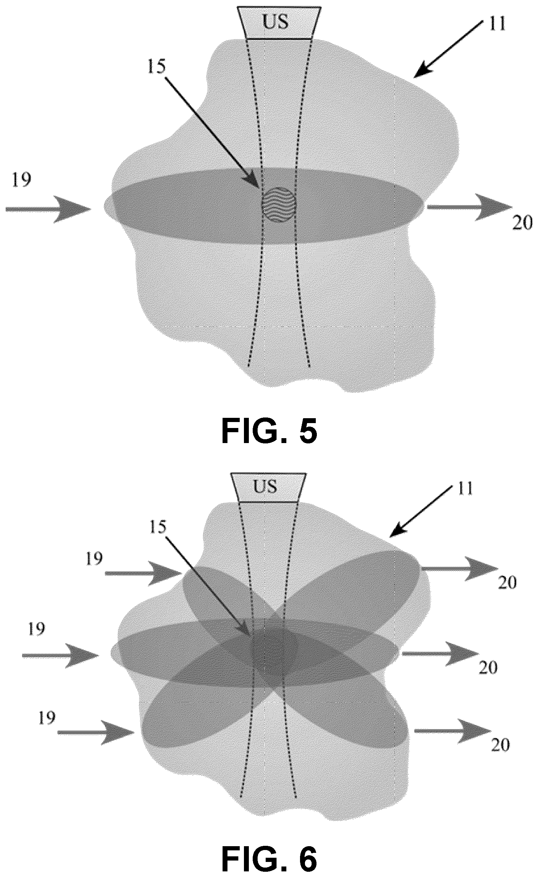

This means that the UOT signal, although primarily localized to the ultrasound focus 15, will also be affected by the absorption along this path as indicated in Fig. 5. Thus, the signal is "smeared" or blurred, which reduces the spatial resolution of the technique, unless more elaborate illumination and detection schemes are used, or unless sophisticated reconstruction algorithms are used to compensate for the blurring effect.

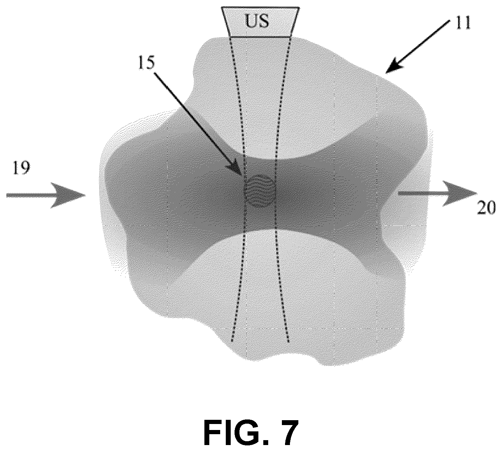

To improve the spatial resolution, and thus reduce image blurring, it advantageous to have multiple light injection positions distributed across the surface, see Fig. 6, or a large area of light illumination, see Fig. 7, to reduce the effect of the image blurring, since multiple light propagation paths at different angles are involved. Even further, having multiple light collection positions distributed across the surface (Fig. 6), or a large area of light collection (Fig. 7), increases the number of light propagation paths at different angles.

A processing unit processes the detected frequency-shifted light from the at least two positions, or from the light source covering an extended area, to provide information of the at least one location inside said tissue site.

The data processing device, processing unit or a computing device may be implemented by special-purpose software (or firmware) run on one or more general-purpose or special-purpose computing devices. In this context, it is to be understood that each "element" or "means" of such a computing device refers to a conceptual equivalent of a method step; there is not always a one-to-one correspondence between elements/means and particular pieces of hardware or software routines. One piece of hardware sometimes comprises different means/elements. For example, a processing unit serves as one element/means when executing one instruction, but serves as another element/means when executing another instruction. In addition, one element/means may be implemented by one instruction in some cases, but by a plurality of instructions in some other cases. Such a software controlled computing device may include one or more processing units, e.g. a CPU ("Central Processing Unit"), a DSP ("Digital Signal Processor"), an ASIC ("Application-Specific Integrated Circuit"), discrete analoge and/or digital components, or some other programmable logical device, such as an FPGA ("Field Programmable Gate Array").

The data processing device, processing unit or computing device may further include a system memory and a system bus that couples various system components including the system memory to the processing unit. The system bus may be any of several types of bus structures including a memory bus or memory controller, a peripheral bus, and a local bus using any of a variety of bus architectures. The system memory may include computer storage media in the form of volatile and/or non-volatile memory such as read only memory (ROM), random access memory (RAM) and flash memory. The special-purpose software may be stored in the system memory, or on other removable/non-removable volatile/non-volatile computer storage media which is included in or accessible to the computing device, such as magnetic media, optical media, flash memory cards, digital tape, solid state RAM, solid state ROM, etc. The processing unit may include one or more communication interfaces, such as a serial interface, a parallel interface, a USB interface, a wireless interface, a network adapter, etc., as well as one or more data acquisition devices, such as an A/D converter. The special-purpose software may be provided to the control unit or data processing device on any suitable computer-readable medium, including a record medium and a read-only memory.

Any method or determinations or calculations described herein may be implemented on a processing unit, a data processing device or a computing device. The computer implementation may be done as a computer program or a software to be executed on a control unit, a data processing device or a computing device.

Any methods or determinations or calculations described herein may be performed by a control unit, a data processing device or a computing device. The processing unit, data processing device or computing device may be connected to the system or be a standalone unit.

A set-up using a ring-shaped light source has been described in Kim et al, Sentinel lymph node detection ex vivo using ultrasound-modulated optical tomography, J Biomed Opt. 2008, 13(2) 020507, and in Kim et al, Ultrasound-modulated optical tomography in reflection-mode with ring-shaped light illumination, J Biomed Opt. 2009, 14(2) 024015, with the effect of reducing shadow effects and unwanted surface modulation. However, these papers do not discuss the optimization of the setup nor do they mention multiple sources and/or detectors.

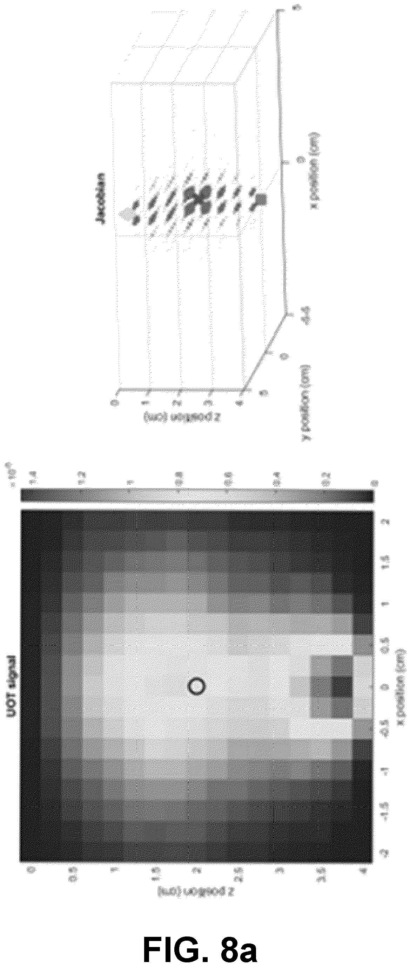

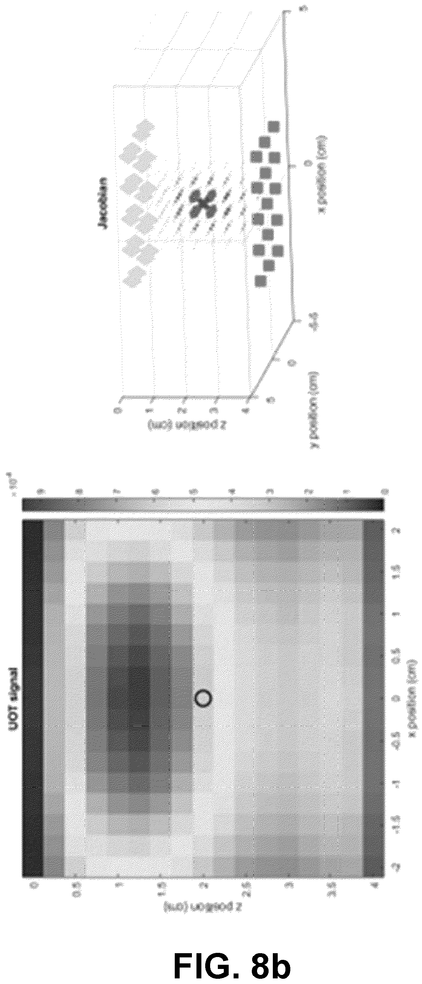

In Figs. 8a and 8b, Monte Carlo simulations of the UOT signal in a cross section throughout a homogeneous tissue sample (left graphs) is illustrated, as well as the Jacobian (right graphs) which through the size of the dots indicates how the absorption at various points in the 3D tissue volume affects the UOT signal at the black circle in the left graphs. The right graphs also indicate the position(s) of the source(s) by diamond(s), detector(s) by square(s) and the location of the ultrasound focus by a cross. In Fig. 8a, there is only one source and detector, and the Jacobian indicates that the UOT signal is heavily affected by the absorption along the path from the source via the ultrasound and to the detector. In Fig. 8b, multiple sources and detectors are used, and in this case, the UOT signal is less dependent on the absorption properties far away from the ultrasound focus (indicated by smaller dots far away from the cross compared to Fig. 8a). Thus, the blurring effect is reduced and the spatial resolution of the UOT technique improved without the need to use additional reconstruction algorithms.

Measurements using multiple light injection positions and/or light collection positions can be performed either simultaneously, or individually and then combining the measurements in post-processing. Furthermore, one can either use multiple sources/detectors or larger source/detectors, or combinations of the two.

Another advantage of having multiple light injection positions distributed across the surface, or a large area of light illumination, is that the combined light fluence in the probed volume will be more even, as shown in the left graph of Fig. 8b compared to that in Fig. 8a. An even light fluence is advantageous since it is easier to detect a small signal variation on a flat background compared to a varying background, since one must in the latter case first compensate for the varying background and this can introduce additional errors. Furthermore, large fluence variations result in a varying signal strength in different parts of the image and places with lower signal have worse signal-to-noise ratios. Thus, any analysis would have to take this into account. In contrast, an even light distribution over the interesting region means that the signal strength is more similar.

Another advantage of having individual multiple light injection positions and/or light collection positions, is that the prior knowledge of multiple light source-detector pairs provides additional information of the light paths that can be used in image reconstruction. This is particular important if the sources are activated one at a time in combination with one detector at a time. By doing this, one can also optimize the amount of frequency-shifted photons compared to the number of carrier photons by predominately analyzing data where there is a large probability that the photons travel via the ultrasound position while moving from the source to the detector. When activating one source and detector at a time, each measurement will have a similar Jacobian as in Fig. 8a), i.e., the UOT signal in one point will depend on the absorption in many other points as mentioned above. For a single measurement, this is a disadvantage. However, when combining the information from several such measurements, one can use the knowledge of where the injection position and light collection position are to determine where the Jacobian is the strongest, and hence use the additional information and perform a tomographic reconstruction of the tissue properties, e.g., similar to what is done in diffuse optical tomography.

Another advantage of distributing light injection over multiple light injection positions distributed across the surface, or a large area of light illumination, is that it is possible to inject more light power into the medium to increase signal to noise, without exceeding safety limits expressed in maximum light power per unit area.

Another advantage of having multiple separate light collection positions is that it is possible to spatially separate the light paths to separate crystals or separate parts of the crystal in the slow-light filter. The crystals could for example be honeycomb shaped or rectangular blocks and packed together next to each other but with some small gaps or other material so that the incoming light in one crystal region is kept in that region through reflections, for example total internal reflections. An additional benefit of this set-up is that it is easier to manufacture several smaller crystals than one large. Thus, one can increase the total detector area that one can use without complicating the crystal growing process. For example, the light can be guided using reflective tubes, such as plastic reflective film tubes, into the cryostat and then into the different crystal regions and out from the regions to the different detectors or different parts of the same detector.

To improve the reconstruction of the image, it is advantageous to perform a scan of the medium to collect data from a 3D region (volume) that is larger than the region of interest of the image. Since detected light has traveled through a large region in the tissue, the signal of the frequency-shifted light depends on the optical properties of the entire region where it is likely that a photon has visited when going from the source(s) to the detector(s). Therefore, it is important to collect data from the entire region to draw better conclusions about the optical properties in the region of interest. This can be achieved either by using an ultrasound transducer that can scan a 3D volume or by translating the setup mechanically. Different information can be gained by moving the ultrasound transducer together or independently from the source/detector. Additional resolution or information can be obtained by examining all collected data simultaneously, instead of limiting the method to examine 2D slices of the data at any one point.

To improve the reconstruction of the image, it is advantageous to use additional knowledge of the optical properties of the tissue that may be obtained by other optical methods that are used in combination with the method of frequency-shifted light. Examples of such methods are time-of-flight optical measurements, frequency-domain photon-migration spectroscopy, or diffuse optical tomography. Either of these methods can be used to provide an average, or low-resolution spatially resolved estimation of the optical properties of the tissue. These measurements can be performed with the same laser or a different laser, e.g., a VCSEL (Vertical-cavity surface-emitting laser). These measurements can be performed with the same wavelengths as the method of frequency-shifted light or at different wavelengths, including multiple wavelengths covering a broad spectral range. The information gathered can be used as complementary information to the method of frequency-shifted light or be used as prior information in the reconstruction of the image in order to improve resolution and contrast.

To even further improve the reconstruction of the image, it is advantageous to use additional knowledge of the composition and structure of the medium, which can be provided by other imaging methods, such as for example X-ray imaging, computed tomography, ultrasound imaging or magnetic resonance imaging (MRI). This can provide complementary or prior information that can be helpful in the reconstruction of the images, e.g., spatial information regarding different tissue regions. Images from methods as those listed above may be used to localize a suspicious region that UOT can help characterize. Alternatively, such images may be used to identify different tissue regions, which may be used as prior information in the UOT reconstruction to reduce its complexity. UOT may also be used to add false-color information to images from the methods listed above. Any MRI data on the molecular composition of tissue may also be used as prior information or combined with the UOT information to provide better accuracy and reduce the uncertainty of the molecular composition.

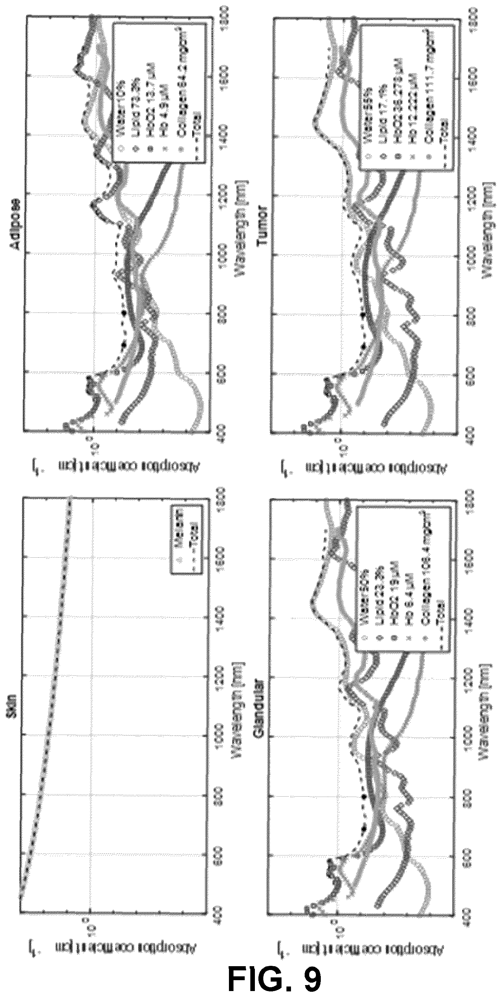

As shown in Fig. 9, the absorption coefficient for different wavelengths varies drastically. Furthermore, the absorption coefficient also varies depending on what type of tissue is examined (skin, adipose, glandular, tumor). One potential aim of UOT is to measure the concentration of tissue constituents (chromophores) such as, but not limited to, water, lipid, deoxygenated and oxygenated blood, collagen, and melanin. However, the UOT signal at one wavelength is only sensitive to the total absorption coefficient. Thus, in order to extract the concentrations of several chromophores one must use at least one wavelength for each relevant chromophore, i.e., for each chromophore that does not have a negligible contribution to the total absorption coefficient at any of the wavelengths used for the UOT measurements. Thus, it is important to extend the use of UOT to multiple wavelengths. Alternatively, one can guess or extract the chromophore concentration for at least one chromophore using literature values, reference measurements potentially using other conventional diagnostic techniques such as X-ray imaging, computed tomography, ultrasound imaging or magnetic resonance imaging, or by using time-of-flight optical measurements, frequency-domain photon-migration spectroscopy, or diffuse optical tomography methods. This could be done with worse spatial resolution than UOT or at shallower depths than UOT, and then the information can be used in the reconstruction of chromophore concentrations based on UOT, which could have increased spatial resolution and/or reach deeper into the tissue.

The primary wavelength range with reasonable absorption coefficients such that deep UOT imaging could be possible would be in the range of 600 nm to around 1100 nm. There are several possible dopants and host crystals covering this wavelength range that could be used as optical filters, including but not limited to: Ho, Er, Eu, Tm, Nd, Dy, Yb, Pr doped into YLF, LaF₃, LiNbO₃, or Li₆YB(O₃)₃.

The UOT method of detecting frequency-shifted light requires a light emitter, e.g., a laser, (12 in Fig. 1) that is well stabilized to a certain wavelength (frequency). The frequency-stabilisation is conventionally achieved by means of an external optical cavity, which is an expensive and sensitive component. It is advantageous to achieve frequency-stabilisation by other means that do not require the use of an external optical cavity. Another means to achieve frequency-stabilisation is by locking the laser frequency to a spectral hole in a crystal, see Julsgaard et al, Understanding laser stabilization using spectral hole burning, Optics Express, Vol 15, No. 18, 11444. Another benefit of stabilizing the laser to a spectral hole is that it can potentially reduce the sensitivity to vibrations. Using a spectral hole instead of a cavity can be cost-beneficial and technologically easier to implement. The spectral hole can be created in the same crystal as used for the slow-light filter, or in a separate crystal. If separate crystals are used, they can either be placed in the same cryostat as the slow-light filter crystal or in different cryostats. The input and output to the spectral hole crystal can be made compact using fibers and, for example, GRIN lenses as both input and output to the crystal.

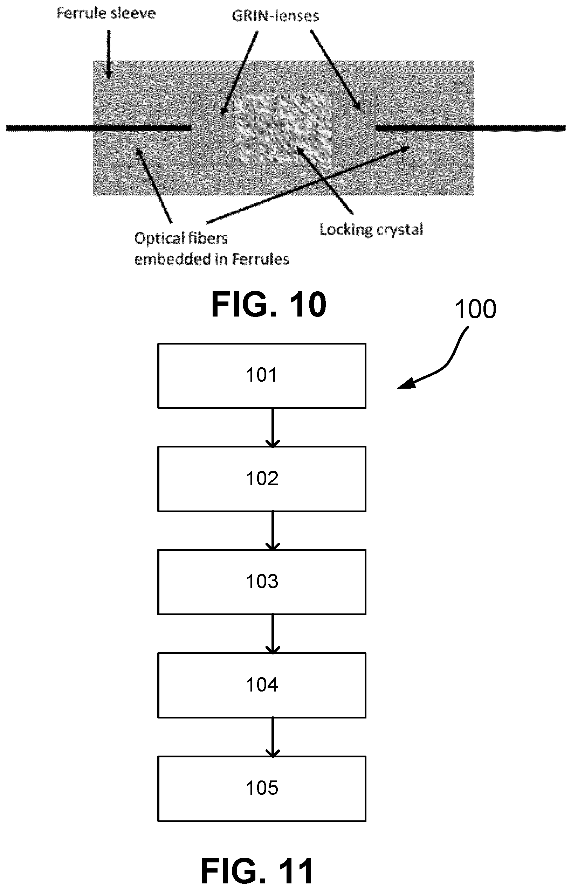

The locking crystal can be arranged in a Ferrule setup as shown in Fig. 10 to minimize the size of the locking setup. If the lifetime of the dopant in the crystal is long, one can shorten it by using RF-fields, electric fields, low or absent of magnetic field, or through laser light.

When performing reconstruction of blood parameters, including for example total blood volume and oxygen saturation, it is advantageous to measure variations in the frequency-shifted light signal that are due to variations caused by changes in blood pressure during the heart beat cycles (photoplethysmography).

When performing image reconstruction and/or data analysis of frequency-shifted light signals, it is advantageous to apply mathematical models including the diffusion equation of light propagation, Monte Carlo (MC) models for light propagation, or artificial neural networks (ANN), convolutional neural networks (CNN), recurrent neural networks (RNN), machine learning (ML), or deep learning. The diffusion equation and MC can be used to easier extract information regarding the spatially resolved optical properties of the medium. They can also be used to compensate for uneven light illumination, by subtracting a simulated frequency-shifted light signal from the experimentally obtained frequency-shifted light signal, thus compensating for an uneven photon flux and highlighting the small frequency-shifted light signal differences due to additional optical scattering or absorption in the tissue.

MC simulations contain information regarding how detected photons have traveled and therefore contain information regarding how absorption in one voxel is expected to affect the frequency-shifted light signal at any voxel which is important for the reconstruction.

ANN, CNN, RNN, and/or ML algorithms can be used in the reconstruction and can potentially detect small UOT signals without compensating for the large variation in photon fluence due to the source(s)/detector(s) position(s). Such algorithms may be trained on simulated data, obtained either through MC simulations or the diffusion equation; on UOT experiments performed on tissue-mimicking phantoms; on UOT experiments performed on healthy volunteers or patients; or on a combination of any of the approaches mentioned above.

In practical implementations of systems for the method of frequency-shifted light with slow-light filters, it is sometimes a problem that fluorescence from the filter material is mixed with the frequency-shifted light. This can compromise the signal, or have the undesired effect that one has to wait before the fluorescence decays between acquisition events (in the order of microseconds to milliseconds). The fluorescence light is sometimes close to, in wavelength, the frequency-shifted light, but not completely overlapping. To remove or reduce the impact of fluorescence, it is advantageous to use other optical filters to attenuate the fluorescence light before it impinges on the light detector. Examples of such filters are, but not limited to, interference filters, color filters, another rare-earth crystal filter, or Rubidium gas cell filters, or other types of gas cell filters.

A method 100, illustrated in Fig. 11, for light measurements in a human or animal.

Directing at least one ultrasound field 101 with at least one ultrasound frequency to at least one location inside a tissue site of said human or animal using at least one ultrasound transducer.

Directing light from at least two light emitters 102 arranged in at least two positions, or from a light emitter covering an extended area, with at least one light frequency into said tissue site of said human or animal. The at least one light emitter is configured for emitting light over an extended area within a wavelength-range of infrared (IR), such as Near infrared (NIR), visible or ultraviolet light.

Supressing the at least one light frequency 103 using at least one optical filter. The Optical filter is arranged before at least one light detector.

Detecting light that is frequency-shifted 104 by the ultrasound frequency by the at least one light detector.

Processing the detected frequency-shifted light 105 from the at least two positions, or the extended area, to provide information of the at least one location inside the tissue site.

The present invention is advantageous to use when there is a medical interest to measure tissue oxygenation deep into the body. For example, the invention may be used in conjunction with ischemic stroke management. In the emergency care setting, it is of importance to rapidly diagnose the presence of an ischemic region in the brain of the patient. A significant portion of ischemic stroke patients undergo thrombectomy to remove the thrombus. In severe cases, the patient cannot be awake during the thrombectomy procedure, which poses the problem that it is not immediately obvious to the surgeon whether the intervention has been successful. In these cases, monitoring of the oxygenation status of the brain would be advantageous as a means of intervention feedback.

Another medical indication where the present invention would be advantageous is for emergency procedures in suspected myocardial infarction, to monitor the oxygenation status of the heart muscle.

Another medical indication where the present invention would be advantageous is in sports medicine, to evaluate oxygen uptake and metabolism in muscles.

Another medical indication where the present invention would be advantageous is in the field of oncology, where the oxygen saturation status may be altered in tumours compared to healthy tissue. For example, there may be necrotic regions in tumours that express low oxygen saturation.

The present invention has been described above with reference to specific examples. However, other examples than the above described are equally possible within the scope of the appended claims . Different method steps than those described above may be provided within the scope of the disclosure. . The different features and steps of the invention may be combined in other combinations than those described. The scope of the disclosure is only limited by the appended patent claims.

The indefinite articles "a" and "an," as used herein in the specification and in the claims, unless clearly indicated to the contrary, should be understood to mean "at least one." The phrase "and/or," as used herein in the specification and in the claims, should be understood to mean "either or both" of the elements so conjoined, i.e., elements that are conjunctively present in some cases and disjunctively present in other cases.

## Claims

1. A system (100) for light measurements in a human or animal, said system comprising:
at least one ultrasound transducer (13) configured to transmit an ultrasonic pulse;
at least one light emitter (12) for emitting light (18a) over an extended area within a wavelength-range of infrared, IR, visible or ultraviolet light;
at least one optical filter (14);
at least one light detector; and
a processing unit;
wherein said ultrasound transducer is configured for directing at least one ultrasound field with at least one ultrasound frequency to at least one location inside a tissue site of said human or animal; said at least one light emitter is configured for directing light from at least two positions with at least one light frequency into said tissue site of said human or animal; said at least one optical filter is arranged before said at least one light detector for suppressing said light frequency but transmitting light with at least one light frequency that is frequency-shifted by said ultrasound frequency; said at least one light detector detecting said light that is frequency-shifted by said ultrasound frequency; and
wherein said processing unit processes said detected frequency-shifted light from said at least two positions, or said extended area, to provide information of said at least one location inside said tissue site;
wherein said detected frequency-shifted light is detected by timing a light measurement to different depth positions of said ultrasonic pulse while illuminating said tissue site with continuous wave, cw, light.

2. The system of claim 1, wherein said at least one light emitter is at least two light sources, such as lasers, arranged to direct light into said tissue site from different discrete positions.

3. The system of claim 1, wherein said at least one light emitter is a single light source extending over an extended area, wherein said single light source is arranged to direct light into said tissue site from said extended area.

4. The system of any of claims 1 to 3, wherein said at least one light detector is at least two light detectors or a spatially resolved detector.

5. The system according to claim 4, wherein said at least one optical filter is at least one crystal, and a separate crystal is arranged before each of said at least two light detectors, or wherein separate regions of a single crystal is associated with each of said at least two detectors or sections of said spatially resolved detector.

6. The system of claim 5, wherein reflective tubes are arranged to guide light to separate parts of said at least one crystal and from each of said separate parts to separate detectors of said at least two detectors or separate sections of said spatially resolved detector.

7. The system of any of claims 1 to 6, wherein said processing unit utilizes information related to a position of said emitted light and a position of said detected frequency-shifted light to provide information of said at least one location inside said tissue site.

8. The system of any of claims 1 to 7, wherein said processing unit provides a reconstruction, such as an image, of said at least one location inside said tissue site.

9. The system according to claims 1 to 8, wherein said at least one ultrasound transducer is configured to scan a 3D volume or be translated.

10. The system of any of claims 1 to 9, wherein said at least one light emitter is configured to emit light corresponding to at least two different wavelengths.

11. The system of any of claims 1 to 10, further comprises at least one of time-of-flight, frequency-domain photon-migration spectroscopy, or diffuse optical tomography to provide optical properties of said tissue.

12. The system of claim 11, wherein said optical properties is used to improve resolution and/or contrast.

13. The system of any of claims 1 to 12, wherein said at least one light emitter is stabilized to an wavelength or frequency by means of an external optical cavity, by locking said wavelength or frequency to a spectral hole in a crystal, or by locking said wavelength or frequency by using a fiber interferometer or fiber cavity.

14. The system of any of claims 1 to 13, wherein a reference is obtained by measuring on at least one separate location, or wherein a reference is obtained through simulations using diffusion equation or Monte Carlo methods, and wherein said reference is removed from said information of said at least one location inside said tissue site.

15. The system of any of claims 1 to 14, wherein said information of said at least one location inside said tissue site is obtained as a function of time and combined with bodily cycles to extract additional information.

## Patentansprüche

1. System (100) für Lichtmessungen bei einem Menschen oder Tier, wobei das System Folgendes umfasst:
mindestens einen Ultraschallwandler (13), der dazu ausgelegt ist, einen Ultraschallimpuls zu übertragen;
mindestens einen Lichtemitter (12) zum Emittieren von Licht (18a) über einen erweiterten Bereich innerhalb eines Wellenlängenbereichs von Infrarot-, IR-, sichtbarem oder ultraviolettem Licht;
mindestens ein optisches Filter (14);
mindestens einen Lichtdetektor; und
eine Verarbeitungseinheit;
wobei der Ultraschallwandler dazu ausgelegt ist, mindestens ein Ultraschallfeld mit mindestens einer Ultraschallfrequenz auf mindestens eine Stelle innerhalb eines Gewebesitus des Menschen oder Tiers zu richten; der mindestens eine Lichtemitter dazu ausgelegt ist, Licht von mindestens zwei Positionen mit mindestens einer Lichtfrequenz in den Gewebesitus des Menschen oder Tiers zu richten; das mindestens eine optische Filter vor dem mindestens einen Lichtdetektor angeordnet ist, um die Lichtfrequenz zu unterdrücken, aber Licht mit mindestens einer Lichtfrequenz zu übertragen, die um die Ultraschallfrequenz frequenzverschoben ist; wobei der mindestens eine Lichtdetektor das Licht detektiert, das um die Ultraschallfrequenz frequenzverschoben ist; und
wobei die Verarbeitungseinheit das detektierte frequenzverschobene Licht von den mindestens zwei Positionen oder dem erweiterten Bereich verarbeitet, um Informationen über die mindestens eine Stelle innerhalb des Gewebesitus bereitzustellen;
wobei das detektierte frequenzverschobene Licht detektiert wird, indem eine Lichtmessung auf verschiedene Tiefenpositionen des Ultraschallpulses zeitlich gesteuert wird, während der Gewebesitus mit Dauerstrichlicht, cw, beleuchtet wird.

2. System nach Anspruch 1, wobei der mindestens eine Lichtemitter mindestens zwei Lichtquellen ist, wie Laser, die angeordnet sind, um Licht von verschiedenen diskreten Positionen in den Gewebesitus zu richten.

3. System nach Anspruch 1, wobei der mindestens eine Lichtemitter eine einzelne Lichtquelle ist, die sich über einen erweiterten Bereich erstreckt, wobei die einzelne Lichtquelle angeordnet ist, um Licht von dem erweiterten Bereich in den Gewebesitus zu richten.

4. System nach einem der Ansprüche 1 bis 3, wobei der mindestens eine Lichtdetektor mindestens zwei Lichtdetektoren oder ein Detektor mit räumlicher Auflösung ist.

5. System nach Anspruch 4, wobei das mindestens eine optische Filter mindestens ein Kristall ist, und ein separater Kristall vor jedem der mindestens zwei Lichtdetektoren angeordnet ist, oder wobei separate Regionen eines einen Kristalls jedem der mindestens zwei Detektoren oder Segmenten des Detektors mit räumlicher Auflösung zugeordnet sind.

6. System nach Anspruch 5, wobei reflektierende Röhren angeordnet sind, um Licht zu separaten Teilen des mindestens einen Kristalls und von jedem der separaten Teile zu separaten Detektoren der mindestens zwei Detektoren oder separaten Segmenten des Detektors mit räumlicher Auflösung zu leiten.

7. System nach einem der Ansprüche 1 bis 6, wobei die Verarbeitungseinheit Informationen in Bezug auf eine Position des emittierten Lichts und eine Position des detektierten frequenzverschobenen Lichts nutzt, um Informationen über die mindestens eine Stelle innerhalb des Gewebesitus bereitzustellen.

8. System nach einem der Ansprüche 1 bis 7, wobei die Verarbeitungseinheit eine Rekonstruktion, wie ein Bild, der mindestens einen Stelle innerhalb des Gewebesitus bereitstellt.

9. System nach den Ansprüchen 1 bis 8, wobei der mindestens eine Ultraschallwandler dazu ausgelegt ist, ein 3D-Volumen zu scannen, oder translatiert zu werden.

10. System nach einem der Ansprüche 1 bis 9, wobei der mindestens eine Lichtemitter dazu ausgelegt ist, Licht zu emittieren, das mindestens zwei unterschiedlichen Wellenlängen entspricht.

11. System nach einem der Ansprüche 1 bis 10, ferner umfassend mindestens eines von Flugzeit, Frequenzdomänen-Photonenmigrationsspektroskopie oder diffuser optischer Tomographie, um optische Eigenschaften des Gewebes bereitzustellen.

12. System nach Anspruch 11, wobei die optischen Eigenschaften verwendet werden, um Auflösung und/oder Kontrast zu verbessern.

13. System nach einem der Ansprüche 1 bis 12, wobei der mindestens eine Lichtemitter mittels eines externen optischen Hohlraums, durch Verriegeln der Wellenlänge oder Frequenz an einem Spektralloch in einem Kristall oder durch Verriegeln der Wellenlänge oder Frequenz unter Verwendung eines Faserinterferometers oder Faserhohlraums auf eine Wellenlänge oder Frequenz stabilisiert wird.

14. System nach einem der Ansprüche 1 bis 13, wobei eine Referenz durch Messen an mindestens einer separaten Stelle erhalten wird, oder wobei eine Referenz durch Simulationen unter Verwendung von Diffusionsgleichung oder Monte-Carlo-Methoden erhalten wird, und wobei die Referenz aus den Informationen der mindestens einen Stelle innerhalb des Gewebesitus entfernt wird.

15. System nach einem der Ansprüche 1 bis 14, wobei die Informationen der mindestens einen Stelle innerhalb des Gewebesitus als eine Funktion der Zeit erhalten und mit Körperzyklen kombiniert werden, um zusätzliche Informationen zu extrahieren.

## Revendications

1. Système (100) destiné à des mesures de lumière chez un être humain ou un animal, ledit système comprenant :
au moins un transducteur ultrasonore (13) configuré pour émettre une impulsion ultrasonore ;
au moins un émetteur de lumière (12) pour émettre de la lumière (18a) sur une zone étendue dans une plage de longueurs d'onde de lumière infrarouge, IR, visible ou ultraviolette ;
au moins un filtre optique (14) ;
au moins un détecteur de lumière ; et
une unité de traitement ;
dans lequel ledit transducteur ultrasonore est configuré pour diriger au moins un champ ultrasonore ayant au moins une fréquence ultrasonore vers au moins un emplacement à l'intérieur d'un site tissulaire dudit être humain ou animal ; ledit au moins un émetteur de lumière est configuré pour diriger de la lumière provenant d'au moins deux positions ayant au moins une fréquence de lumière vers ledit site tissulaire dudit être humain ou animal ;
ledit au moins un filtre optique est disposé en amont dudit au moins un détecteur de lumière pour supprimer ladite fréquence lumineuse, mais transmettre de la lumière ayant au moins une fréquence lumineuse décalée en fréquence de ladite fréquence ultrasonore ; ledit au moins un détecteur de lumière détectant ladite lumière décalée en fréquence de ladite fréquence ultrasonore ; et
dans lequel ladite unité de traitement traite ladite lumière décalée en fréquence détectée à partir desdites au moins deux positions, ou de ladite zone étendue, pour fournir des informations dudit au moins un emplacement à l'intérieur dudit site tissulaire ;
dans lequel ladite lumière décalée en fréquence détectée est détectée en synchronisant une mesure de lumière à différentes positions de profondeur de ladite impulsion ultrasonore tout en éclairant ledit site tissulaire avec une lumière à onde continue, cw.

2. Système selon la revendication 1, dans lequel ledit au moins un émetteur de lumière consiste en au moins deux sources de lumière, telles que des lasers, conçues pour diriger de la lumière vers ledit site tissulaire à partir de différentes positions discrètes.

3. Système selon la revendication 1, dans lequel ledit au moins un émetteur de lumière est une source de lumière unique s'étendant sur une zone étendue, dans lequel ladite source de lumière unique est conçue pour diriger de la lumière vers ledit site tissulaire à partir de ladite zone étendue.

4. Système selon l'une quelconque des revendications 1 à 3, dans lequel ledit au moins un détecteur de lumière consiste en au moins deux détecteurs de lumière ou un détecteur spatialement résolu.

5. Système selon la revendication 4, dans lequel ledit au moins un filtre optique est au moins un cristal, et un cristal séparé est agencé en amont de chacun desdits au moins deux détecteurs de lumière, ou dans lequel des régions séparées d'un cristal unique sont associées à chacun desdits au moins deux détecteurs ou sections dudit détecteur spatialement résolu.

6. Système selon la revendication 5, dans lequel des tubes réfléchissants sont agencés pour guider la lumière vers des parties séparées dudit au moins un cristal et de chacune desdites parties séparées vers des détecteurs séparés desdits au moins deux détecteurs ou sections séparées dudit détecteur spatialement résolu.

7. Système selon l'une quelconque des revendications 1 à 6, dans lequel ladite unité de traitement utilise des informations relatives à une position de ladite lumière émise et à une position de ladite lumière décalée en fréquence détectée pour fournir des informations dudit au moins un emplacement à l'intérieur dudit site tissulaire.

8. Système selon l'une quelconque des revendications 1 à 7, dans lequel ladite unité de traitement fournit une reconstruction, telle qu'une image, dudit au moins un emplacement à l'intérieur dudit site tissulaire.

9. Système selon les revendications 1 à 8, dans lequel ledit au moins un transducteur ultrasonore est configuré pour balayer un volume 3D ou être translaté.

10. Système selon l'une quelconque des revendications 1 à 9, dans lequel ledit au moins un émetteur de lumière est configuré pour émettre de la lumière correspondant à au moins deux longueurs d'onde différentes.

11. Système selon l'une quelconque des revendications 1 à 10, comprenant en outre au moins un élément parmi la spectroscopie à temps de vol, la spectroscopie à migration de photons dans le domaine fréquentiel ou la tomographie optique diffuse pour fournir des propriétés optiques dudit tissu.

12. Système selon la revendication 11, dans lequel lesdites propriétés optiques sont utilisées pour améliorer la résolution et/ou le contraste.

13. Système selon l'une quelconque des revendications 1 à 12, dans lequel ledit au moins un émetteur de lumière est stabilisé à une longueur d'onde ou à une fréquence au moyen d'une cavité optique externe, en bloquant ladite longueur d'onde ou fréquence à un trou spectral dans un cristal, ou en bloquant ladite longueur d'onde ou fréquence à l'aide d'un interféromètre à fibre ou d'une cavité à fibre.

14. Système selon l'une quelconque des revendications 1 à 13, dans lequel une référence est obtenue en mesurant sur au moins un emplacement séparé, ou dans lequel une référence est obtenue par des simulations en utilisant l'équation de diffusion ou des méthodes de Monte Carlo, et dans lequel ladite référence est éliminée desdites informations dudit au moins un emplacement à l'intérieur dudit site tissulaire.

15. Système selon l'une quelconque des revendications 1 à 14, dans lequel lesdites informations dudit au moins un emplacement à l'intérieur dudit site tissulaire sont obtenues en fonction du temps et combinées à des cycles corporels afin d'extraire des informations supplémentaires.
